(19) Europäisches Patentamt
European Patent Office
Office européen des brevets

(11) Publication number: 0 541 312 A1

(12) # EUROPEAN PATENT APPLICATION

(21) Application number: 92309999.8

(51) Int. Cl.[5]: C07C 43/04, C07C 41/44

(22) Date of filing: 02.11.92

(30) Priority: 04.11.91 US 787210

(43) Date of publication of application: 12.05.93 Bulletin 93/19

(84) Designated Contracting States: DE FR GB

(71) Applicant: TEXACO CHEMICAL COMPANY
3040 Post Oak Boulevard
Houston, Texas 77056 (US)

(72) Inventor: Sanderson, John Ronald
15290 Faubion Trail
Leander, TX 78641 (US)
Inventor: Knifton, John Frederick
10900 Catskill Trail
Austin, TX 78726 (US)
Inventor: Stockton, Melvin Ernest
459 Oak Crest Lane
Georgetown, TX 78628 (US)

(74) Representative: Green, Mark Charles et al
Urquhart-Dykes & Lord, 91 Wimpole Street
London W1M 8AH (GB)

(54) **Catalytic decomposition of impurities in methyl tertiary butyl ether.**

(57) Motor-fuel grade methyl tertiary butyl ether which is prepared by reacting methanol with a tertiary butyl alcohol reaction product contaminated with residual amounts of tertiary butyl hydroperoxide and ditertiary butyl peroxide can be effectively catalytically treated under mild conversion conditions including a temperature of about 180° to 280°C. with an iron oxide catalyst in order to substantially convert the peroxide contaminants and to thereby provide a treated methyl tertiary butyl ether product substantially free from contaminating quantities of such peroxides.

EP 0 541 312 A1

This invention relates to the catalytic purification of methyl tertiary butyl ether prepared by the reaction of methanol with t-butyl alcohol contaminated with trace quantities of t-butyl hydroperoxide and ditertiary butyl peroxide. More particularly, this invention relates to a method for the removal of residual contaminating quantities of tertiary butyl hydroperoxide and ditertiary butyl peroxide from a methyl tertiary butyl ether feedstock which is prepared by the reaction of methanol with tertiary butyl alcohol and is useful as an octane-enhancing component for motor fuels. In accordance with the present invention the peroxide-contaminated feedstock is brought into contact with an iron oxide catalyst in order to substantially selectively decompose both the tertiary butyl hydroperoxide and the ditertiary butyl peroxide.

It is known to those skilled in the art that ethers, including unsymmetrical ethers, may be prepared by reacting an alcohol with another alcohol to form the desired product. The reaction mixture, containing catalyst and/or condensing agent may be separated and further treated to permit attainment of the desired product. Such further treatment commonly includes one or more distillation operations.

Methyl tert-butyl ether is finding increasing use as a blending component in high octane gasoline as the current gasoline additives based on lead and manganese are phased out. Currently commercial processes for the manufacture of methyl tert-butyl ether are based upon the liquid-phase reaction of isobutylene and methanol (Eq. 1), catalyzed by a cationic ion-exchange resin (see, for example: Hydrocarbon Processing, Oct. 1984, p. 63; Oil and Gas J., Jan. 1, 1979, p. 76; Chem. Economics Handbook-SRI, Sept. 1986, p. 543-7051P). The cationic ion-exchange resins used in MTBE synthesis normally have the sulphonic acid functionality (see: J. Tejero, J. Mol. Catal., 42 (1987) 257; C. Subramamam et al., Can. J. Chem. Eng., 65 (1987) 613).

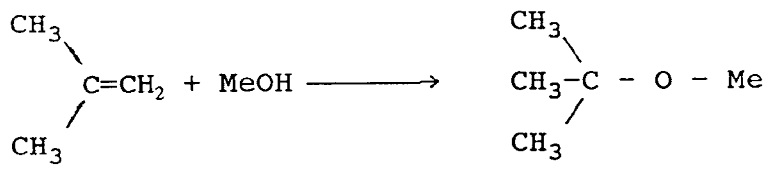

(Eq. 1)

With the expanding use of MTBE as an acceptable gasoline additive, a growing problem is the availability of raw materials. Historically, the critical raw material is isobutylene (Oil and Gas J., June 8, 1987, p. 55). It would be advantageous, therefore, to have a process to make MTBE that does,not require isobutylene as a building block. It would be advantageous to have an efficient process for making MTBE by reaction of methanol with tertiary butyl alcohol, since t-butanol (TBA) is readily available commercially through isobutane oxidation.

In U. S. Patent No. 4,144,138 (1979) to Rao et al., there is disclosed a method for recovering methyl tertiary butyl ether from etherification reaction effluent by azeotropic distillation to recover methanol-ether azeotrope overhead which is water-washed to give pure ether raffinate, the matter being azeotropically distilled to yield ether-methanol overhead which is recycled to water washing.

The preparation of methyl tert-butyl ether from methyl and tert-butyl alcohols is discussed in S. V. Rozhkov et al., Prevrashch Uglevodorodov, Kislotno-Osnovn. Geterogennykh Katal. Tezisy Dokl. Vses Konf., 1977, 150 (C. A. 92:58165y). Here the TBA and methanol undergo etherification over KU-2 strongly acidic sulfopolystyrene cation-exchangers under mild conditions. This reference contains data on basic parameters of such a process. It is also pointed out that, although a plant for etherification over, cation exchangers does not present any major problems, considerations include the fact that recycling large amounts of tert-butyl alcohol and methanol, as well as isobutylene, causes the scheme to be somewhat more expensive. Also, the progress of the reaction over cation exchangers is usually complicated by various adsorption and diffusion factors, by swelling phenomena, and by the variable distribution of the components between the solution and ion-exchanger phase. Furthermore, said acidic cation-exchangers with an organic (polystyrene or polymethacrylate) backbone generally have a very limited stability range with regard to operating temperatures, with temperatures above 120°C. normally leading to irreversible destruction of the resin and loss of catalytic activity.

Sanderson U. S. Patent No. 4,900,850 discloses a method for removing ditertiary butyl peroxide from t-butyl alcohol by water extraction.

Sanderson et al. also disclose catalytic methods for the purification of t-butyl alcohol contaminated with residual quantities of tertiary butyl hydroperoxide and ditertiary butyl peroxide using catalysts such as catalysts composed of mixtures of nickel, copper, chromia and iron (U. S. Patent No. 4,704,482), catalysts composed of mixtures of iron, copper, chromia and cobalt (U. S. Patent No. 4,705,903), catalysts composed of mixtures of nickel, copper, chromium and barium (U. S. Patent No. 4,873,380), or catalysts composed of metals selected from group VIB or VIIIB of the Periodic Table (U. S. Patent No. 4,742,179).

It has heretofore been proposed, as shown, for example, by Grane U. S. Patent No. 3,474,151 to thermally decompose tertiary butyl hydroperoxide and ditertiary butyl peroxide to form tertiary butyl alcohol. The thermal decomposition must be conducted with care, as pointed out by Grane, in that tertiary butyl alcohol will start

to dehydrate at a temperature of about 230°C (450°F) and in that the dehydration becomes rapid at temperatures above about 250°C (475°F). Moreover, the product from the thermal decomposition normally contains a minor amount of tertiary butyl hydroperoxide and ditertiary butyl peroxide which have an adverse effect upon the quality of motor fuels and must be substantially completely removed if the tertiary butyl alcohol is to be fully effective. Grane proposes to accomplish this thermally by heating tertiary butyl alcohol containing small quantities of such peroxides at a temperature of 190°-250°C (375-475°F) for a period of 1 to 10 minutes.

This concept was expanded upon by Grane et al. in U. S. Patent Nos. 4,294,999 and 4,296,262 to provide integrated processes wherein, starting with isobutane, motor-fuel grade tertiary butyl alcohol was prepared by the oxidation of isobutane (e.g., in the presence of a solubilized molybdenum catalyst) to produce a mixture of tertiary butyl alcohol and tertiary butyl hydroperoxide from which a fraction rich in tertiary butyl hydroperoxide could be recovered by distillation. This stream, after being debutanized was subjected to thermal decomposition under pressure at a temperature of less than 150°C (300°F) for several hours to significantly reduce the concentration of the tertiary butyl hydroperoxide. However, the product of this thermal decomposition step still contained residual tertiary butyl hydroperoxide, most of which was thereafter removed by a final thermal treatment of the contaminated tertiary butyl hydroperoxide in the manner taught by Grane U. S. Patent No. 3,474,151.

Thus, the removal of trace quantities of tertiary butyl hydroperoxide from motor grade tertiary butyl alcohol has received appreciable attention. However, little appears to have been published concerning the removal of trace quantities of ditertiary butyl peroxide, the more refractory of the two peroxides. This may be explainable both because ditertiary butyl peroxide is not always present in trace quantities in motor grade tertiary butyl alcohol (its presence or absence being a function of the reaction conditions used in oxidizing the isobutane starting material) and because, when present, it is present in significantly lower amounts. For example, after decomposition of the major amount of tertiary butyl hydroperoxide formed by the oxidation of isobutane, the tertiary butyl hydroperoxide residual content will normally be about 0.1 to about 1 wt.%, based on the tertiary butyl alcohol, while the residual ditertiary butyl peroxide content, if any, will only be about 0.1 to 0.5 wt.%.

It has also been proposed to remove the residual hydroperoxide contaminants from tertiary butyl alcohol through the use of a heterogeneous cobalt oxide catalyst containing a copper oxide promoter as shown, for example, by Coile U. S. Patent No. 4,059,598. Allison et al. in U. S. Patent No. 3,505,360 have more generically taught that alkenyl hydroperoxides can be decomposed catalytically through the use of a catalyst based on a metal or compound of a metal of group IV-A, V-A or VI-A.

In West German DE 3248465-A a two-step process is disclosed wherein isobutane is oxidized noncatalytically with air to a conversion of about 48-90% to form the corresponding hydroperoxide, which is then catalytically decomposed under hydrogenation conditions in the presence of a supported catalyst such as palladium, platinum, copper, rhenium, ruthenium or nickel to form tertiary butyl alcohol. The decomposition product obtained using 1.3% palladium on lithium spinel as a catalyst contained significant quantities of acetone, water and methanol.

When isobutane is reacted with molecular oxygen the principal products of the reaction are tertiary butyl alcohol and tertiary butyl hydroperoxide. However, minor amounts of other peroxides, including ditertiary butyl peroxide are also formed. Generally speaking, from about 10 to about 100 parts of tertiary butyl hydroperoxide are formed per part of ditertiary butyl peroxide. Minor quantities of other contaminants are also formed.

A listing of the components present in a representative reaction product, and their nominal boiling points is given in Table A.

TABLE A

| Component | NBP (°C) |
|---|---|
| Isobutane | -11.7 |
| Methyl formate | 31.8 |
| Acetone | 56.3 |
| Isobutylene oxide | 60.0 |
| Isobutyraldehyde | 64.1 |
| Methanol | 64.7 |
| Methyl-t-buthyl peroxide | 74.2 |
| Isopropyl alcohol | 82.3 |
| Tertiary butyl alcohol | 82.4 |
| Ditertiary butyl peroxide | 111.0 |
| t-butyl-i-pr-peroxide | 124.0 |
| Tertiary butyl formate | 163.8 |

The minor by-products are sometimes difficult to remove. For example, tertiary butyl formate has a higher boiling point than ditertiary butyl hydroperoxide but tends to distill overhead, which suggests that it forms a minimum boiling azeotrope with another component or components.

When tertiary butyl alcohol is prepared by the catalytic decomposition of tertiary butyl hydroperoxide or by the catalytic reaction of tertiary butyl hydroperoxide with an olefin such as propylene, the tertiary butyl alcohol will be contaminated with residual quantities of tertiary butyl hydroperoxide and ditertiary butyl peroxide, as explained above, and if tertiary butyl alcohol prepared in this manner is used as a feedstock for the direct preparation of MTBE by reaction with methanol, the tertiary butyl hydroperoxide and ditertiary butyl peroxide impurities will remain with the MTBE, and must be removed to the lowest level that is feasibly possible if the MTBE is to be used in the preparation of fuel for internal combustion engines. The object of the present invention is to provide a method to effectively remove contaminating peroxides.

It has been surprisingly discovered in accordance with the present invention that methyl tertiary butyl ether (MTBE) that is contaminated with residual amounts of tertiary butyl hydroperoxide and ditertiary peroxide can be effectively treated for the substantially complete removal of such peroxide contaminants by bringing the contaminated MTBE into contact with an iron oxide catalyst at a superatmospheric pressure at a temperature of 120° to 280°C.

It has been further discovered that most of the decomposition products that are formed are decomposition products that can be removed from the MTBE with comparative ease if the treatment with the iron oxide catalyst is conducted prior to distillation. The principle decomposition products are tertiary butyl alcohol (which does not adversely affect the octane rating of the MTBE product), and methanol and acetone, both of which can be removed from MTBE by distillation.

The results obtained with the process of the present invention are surprising and unexpected in several respects. Catalytic or thermal decomposition of tertiary butyl hydroperoxide and ditertiary butyl peroxide can result in the formation of acetone and methanol and other lower value by-products . Thus, the favorable effect on the quality of motor fuel grade.tertiary butyl alcohol that is obtained by the substantially complete.elimination of the two peroxides will be largely counterbalanced if the decomposition product produces significant quantities of acetone.

Thus, the provision of the process of the present invention wherein a motor-fuel grade MTBE feedstock containing contaminating quantities of both ditertiary butyl peroxide and tertiary butyl hydroperoxide, is catalytically treated for the decomposition of the peroxides so that they are substantially completely removed without significantly adding to the level of contamination due to the formation of acetone, methanol and other low value by-products constitutes a distinct advantage.

STARTING MATERIALS

The starting materials for the process of the present invention include a motor-fuel grade MTBE feedstock obtained in the manner described above that is contaminated with tertiary butyl hydroperoxide and ditertiary butyl peroxide.

The motor-fuel grade MTBE feedstock will contain contaminating quantities of teritary butyl hydroperoxide and ditertiary butyl peroxide. The levels of contamination of such materials are such that the tertiary butyl alcohol will normally contain, prior to treatment, from about 0.1 to about 5 wt.% of tertiary butyl hydroperoxide and from about 0.1 to about 5 wt.% of ditertiary butyl peroxide. Minor quantities of other contaminants may also be present.

Catalytic Treatment of Methyl Tertiary Butyl Ether

In accordance with the present invention, an MTBE feedstock, as above described, is brought into contact with an iron oxide catalyst of the present invention under reaction conditions correlated to catalytically convert both the tertiary butyl hydroperoxide and ditertiary butyl peroxide contaminants in the tertiary butyl alcohol feedstock to tertiary butyl alcohol with not more than a minor increase in the level of contamination of the acetone, methanol and other lower value by-products.

The reaction may be conducted batchwise in an autoclave using powdered catalyst or may be conducted on a continuous basis by passing the tertiary butyl alcohol through a reactor containing a bed of a catalyst of the present invention under reaction conditions including a temperature within the range of about 100° to about 280°C and, more preferably, from about 120° to about 180°C. The reaction is preferably conducted at 1380 to 13800 kPa gauge pressure (200 to 2000 psig), although higher pressures may be used if desired. When the reaction is conducted batchwise, contact time may suitably be from 0.5 to 4 hours. When the reaction is conducted on a continuous basis, the MTBE should be passed over the bed of catalyst at a liquid hourly space velocity of 0.25 to 5.

The reaction product, after distillation to remove methanol, acetone, etc., is suitable for use as an octane-enhancing component of motor fuel, such as gasoline.

WORKING EXAMPLES

Reactor

The reactor was a stainless steel tube (13mm x 736mm) which was electrically heated. Clean glass beads were charged to the reactor to aid in heat transfer when no catalyst was used. Liquid feed was pumped into the bottom of the reactor. Pressure regulation was with a Skinner Uni-Flow valve and a Foxboro controller. The liquid feed was pumped with a Ruska dual drive pump.

Feed

The "pseudo-recycle" feed for the decomposition reactor was 10% water, 51% methanol, 35% TBA, 1.0% MTBE, and 2.5% DTBP.

Catalyst Preparation

One kg Aldrich iron chips (cat# 26,794-5) were placed in a beaker and covered with 2% sulfuric acid. Air was blown through the chps at about 200 cc/hour at ambient temperature overnight. The chips were then filtered and let stand at ambient temperature for several weeks. The chips were completely covered with a rusty-brown color of iron oxide(s).

Results

In the attached Tables, Runs 1-8 are ones using the catalyst of the invention, and Runs 9-16 ones wherein glass beads are charged in the reactor for comparison. A and B are analytical values for each feedstock.

TABLE 1-A

| TEST | A | 1 | 2 | 3 | 4 |
|---|---|---|---|---|---|
| Catalyst | | $Fe_2O_3$ on Fe Chips | $Fe_2O_3$ on Fe Chips | $Fe_2O_3$ on Fe Chips | $Fe_2O_3$ on Fe Chips |
| Reactor (cc) | | 100 | 100 | 100 | 100 |
| Pressure (psig)* | | 500 | 500 | 500 | 500 |
| Feed rate (cc/hr) | | 100 | 100 | 100 | 100 |
| Temperature (°C) | | 120 | 140 | 160 | 180 |
| Time on stream (hr) | | 4 | 4 | 4 | 4 |
| Space vel. (cc/cc) | | 1.0 | 1.0 | 1.0 | 1.0 |
| DTBP Conv. (%) | | 12.8 | 38.4 | 97.8 | 99.9 |
| TBA Conversion (%) | | 0.0 | 0.0 | 0.0 | 0.0 |
| IC4 | 0.004 | 0.002 | 0.002 | 0.003 | 0.009 |
| MEOH/MF | 55.631 | 55.690 | 55.630 | 55.398 | 55.111 |
| Acetone | 0.000 | 0.078 | 0.343 | 1.172 | 1.522 |
| MTBE | 1.213 | 1.145 | 1.171 | 1.165 | 1.170 |
| TBA | 40.024 | 40.477 | 40.892 | 41.820 | 41.573 |
| DTBP | 2.765 | 2.411 | 1.702 | 0.062 | 0.004 |
| Metals (ppm) | | | | | |
| Fe | <1 | <1 | <1 | <1 | <1 |
| Acid No. (mg/g) | 0.01 | 0.03 | 0.03 | 0.06 | 0.03 |

*500 psig = 3448 kPa gauge pressure

IC4 = Isobutylene

TABLE 1-B

| TEST | A | 5 | 6 | 7 | 8 |
|---|---|---|---|---|---|
| Catalyst | | Fe2O3 on Fe Chips | Fe2O3 on Fe Chips | Fe2O3 on Fe Chips | Fe2O3 on Fe Chips |
| Reactor (cc) | | 100 | 100 | 100 | 100 |
| Pressure (psig) | | 500 | 500 | 500 | 500 |
| Feed rate (cc/hr) | | 200 | 200 | 200 | 200 |
| Temperature (°C) | | 120 | 140 | 160 | 180 |
| Time on stream (hr) | | 4 | 4 | 4 | 4 |
| Space vel. (cc/cc) | | 2.0 | 2.0 | 2.0 | 2.0 |
| DTBP Conv. (%) | | 6.3 | 28.9 | 95.8 | 100.0 |
| TBA Conversion (%) | | 0.0 | 0.0 | 0.0 | 0.0 |
| IC4 | 0.004 | 0.002 | 0.003 | 0.003 | 0.009 |
| MEOH/MF | 55.631 | 55.667 | 55.384 | 55.182 | 55.053 |
| Acetone | 0.000 | 0.057 | 0.592 | 1.326 | 1.922 |
| MTBE | 1.213 | 1.169 | 1.183 | 1.157 | 1.186 |
| TBA | 40.024 | 40.270 | 40.544 | 41.629 | 41.200 |
| DTBP | 2.765 | 2.591 | 1.966 | 0.115 | 0.000 |
| Metals (ppm) | | | | | |
| Fe | <1 | <1 | <1 | 2.0 | <1 |
| Acid No. (mg/g) | 0.01 | 0.01 | 0.02 | 0.03 | 0.04 |

TABLE 1-C

| TEST | B | 9 | 10 | 11 | 12 |
|---|---|---|---|---|---|
| Catalyst | | Glass Beads | Glass Beads | Glass Beads | Glass Beads |
| Reactor (cc) | | 100 | 100 | 100 | 100 |
| Pressure (psig) | | 500 | 500 | 500 | 500 |
| Feed rate (cc/hr) | | 100 | 100 | 100 | 100 |
| Temperature (°C) | | 130 | 140 | 150 | 160 |
| Time on stream (hr) | | 4 | 4 | 4 | 4 |
| Space vel. (cc/cc) | | 1.0 | 1.0 | 1.0 | 1.0 |
| DTBP Conv. (%) | | 0.0 | 6.2 | 65.4 | 97.5 |
| TBA Conversion (%) | | 3.7 | 4.6 | 2.2 | 0.0 |
| IC4 | 0.004 | 0.001 | 0.001 | 0.002 | 0.004 |
| MEOH/MF | 56.015 | 56.892 | 57.297 | 57.003 | 56.040 |
| Acetone | 0.008 | 0.000 | 0.112 | 0.863 | 1.113 |
| MTBE | 1.194 | 1.447 | 1.493 | 1.509 | 1.288 |
| TBA | 39.912 | 38.431 | 38.094 | 39.029 | 40.763 |
| DTBP | 2.792 | 2.805 | 2.619 | 0.965 | 0.069 |
| Metals (ppm) | | | | | |
| Fe | <1 | <1 | <1 | <1 | <1 |
| Acid No. (mg/g) | 0.01 | 0.01 | 0.02 | 0.02 | 0.02 |

TABLE 1-D

| TEST | B | 13 | 14 | 15 | 16 |
|---|---|---|---|---|---|
| Catalyst | | Glass Beads | Glass Beads | Glass Beads | Glass Beads |
| Reactor (cc) | | 100 | 100 | 100 | 100 |
| Pressure (psig) | | 500 | 500 | 500 | 500 |
| Feed rate (cc/hr) | | 200 | 200 | 200 | 200 |
| Temperature (°C) | | 130 | 140 | 150 | 160 |
| Time on stream (hr) | | 4 | 4 | 4 | 4 |
| Space vel. (cc/cc) | | 2.0 | 2.0 | 2.0 | 2.0 |
| DTBP Conv. (%) | | 8.0 | 19.7 | 29.9 | 56.0 |
| TBA Conversion (%) | | 0.0 | 0.0 | 0.0 | 0.0 |
| IC4 | 0.004 | 0.003 | 0.003 | 0.004 | 0.002 |
| MEOH/MF | 56.015 | 55.721 | 54.801 | 55.050 | 55.409 |
| Acetone | 0.008 | 0.076 | 0.150 | 1.050 | 1.734 |
| MTBE | 1.194 | 1.260 | 1.167 | 1.217 | 1.228 |
| TBA | 39.912 | 39.977 | 41.212 | 40.266 | 40.825 |
| DTBP | 2.792 | 2.570 | 2.243 | 1.956 | 1.229 |
| Metals (ppm) | | | | | |
| Fe | <1 | <1 | <1 | <1 | <1 |
| Acid No. (mg/g) | 0.01 | 0.01 | 0.01 | 0.01 | 0.02 |

It is noted that decomposition of the peroxide is faster in the presence of the catalyst of this invention. Compare, for example, Test 7 (Table 1-B) with a DTBP conversion of 95.8%. Under identical conditions with no catalyst in Test 16 a 56.0% DTBP conversion is observed (Table 1-D).

In general, the higher the temperature of the decomposition, the more undesirable by-products such as acetone increase. According to the present invention, it is possible to decompose using the catalyst at a lower temperature and therefore obtain less acetone and other by-products. For example, at 140°C. (Test 6, Table 1-B) a 28.9% DTBP conversion is observed and a wt.% acetone of 1.183. Without catalyst, a conversion of

29.9% is observed at 150°C. (Test 15, Table 1-D) and a wt.% acetone of 1.217.

## Claims

1. A method for the treatment of methyl tertiary butyl ether feedstock contaminated with residual quantities of tertiary butyl hydroperoxide and ditertiary butyl peroxide, characterised by
   contacting said feedstock in a reaction zone with an iron oxide catalyst at a temperature of 120° to 280°C for a period of time sufficient to substantially selectively decompose said tertiary butyl hydroperoxide and said ditertiary butyl peroxide to tertiary butyl alcohol.

2. A method as claimed in Claim 1 wherein the reaction is conducted at a temperature of about 120° to about 180°C.

3. A method as claimed in Claim 1 or Claim 2 characterised in that the reaction is conducted at a pressure of from 0 to 13800 kPa gauge pressure.

4. A tertiary butyl ether product produced by a method as claimed in any one of Claims 1 to 3 characterised by containing not more than about 100 ppm of tertiary butyl hydroperoxide, not more than about 100 ppm of ditertiary butyl peroxide and not more than about 0.5 wt.% of isobutylene.

European Patent Office

# EUROPEAN SEARCH REPORT

Application Number

EP 92 30 9999

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | US-A-4 705 903 (J. R. SANDERSON ET AL) * claims * | 1-3 | C07C43/04<br>C07C41/44 |
| D,A | US-A-4 704 482 (J. R. SANDERSON ET AL) * claims * | 1-3 | |
| D,A | US-A-4 742 179 (J. R. SANDERSON ET AL) * claims * | 1-3 | |
| X | 'KATALOG 15 CHEMIKA-BIOCHEMIKA 1986/87' 1986 , FLUKA , BUCHS, SWITZERLAND * page 268, No. 20249 * | 4 | |
| P,A | US-A-5 159 122 (J. R. SANDERSON ET AL) * claims; figure * | 1-4 | |

TECHNICAL FIELDS SEARCHED (Int. Cl.5 )

C07C

The present search report has been drawn up for all claims

| Place of search | Date of completion of the search | Examiner |
|---|---|---|
| THE HAGUE | 02 FEBRUARY 1993 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)